# EUROPEAN PATENT APPLICATION

(11) **EP 2 636 651 A1**
(43) Date of publication of application: **11.09.2013**
(21) Application number: 13156311.6
(22) Date of filing: 22.02.2013
(51) Int. Cl.: C02F 11/04, C02F 3/28, C05F 17/00, C02F 3/00

(54) **System for processing biomass**

(30) Priority: 06.03.2012 NL 1039443
(71) Applicant: Lely Patent N.V., 3147 PA Maassluis (NL)
(72) Inventor: Hazewinkel, Jacob Hendrik Obbo, 3147 PA Maassluis (NL)
(74) Representative: Corten, Maurice Jean F.M.

(57) **Abstract**

The invention relates to a system for processing biomass using anaerobic digestion, comprising an anaerobic acidification reactor (1) comprising an inlet (11) for biomass and an outlet (12) for acidified biomass, an anaerobic settling chamber (2) comprising an inlet (21) for receiving the acidified biomass from the anaerobic acidification reactor (1), wherein an acidified liquid biomass and a sludge biomass is formed. The anaerobic settling chamber (2) comprises a first outlet (22) for the acidified liquid biomass, a second outlet (23) for the sludge biomass and a third outlet (24) for gaseous biogas effluent. The system further comprises an anaerobic methane synthesis reactor (3) for anaerobic microbial conversion of acidified liquid biomass into biogas and processed liquid biomass, comprising an inlet (31) for receiving the acidified liquid biomass and a first outlet (32) for gaseous biogas effluent and a second outlet (33) for the processed liquid biomass.

## Description

### TECHNICAL FIELD

The invention relates to a system for processing biomass using anaerobic digestion.

### PRIOR ART

In the Netherlands, the average annual production of biomass is approximate1y 30 Mt dry matter of which nearly 28 Mt is produced in agriculture. This tonnage represents an energy content of 475 PJ of renewable energy each year equal to 15 billion m³ natural gas. In comparison, in the Netherlands, the use of natural gas was 1400 PJ in 2008. Up to know, the energy content of biomass is not optimally used.

Conversion of non-used biomass, such as agricultural and forestry waste and organic waste streams of the primarily used biomass into high-grade products, for example natural gas, can significantly contribute to the amount of sustainable energy, or green energy, available for energy consumption and, accordingly, significantly contribute to reduction of greenhouse gasses such as CO₂.

Efficient conversion of the biomass produced in agriculture, for instance into methane or natural gas, could provide a renewable potential alternative for a substantial part of the natural gas consumption.

Conversion of organic waste streams, and especially manure, into methane has been used for decades. The most commonly used method basically comprises a large air-sealed holder in which manure is collected and allowed to ferment, i.e. convert or digest, carbon based or organic materials into methane, generally for 30 to 40 days. The resulting (bio)gas generally comprises approximately 40% CO₂, 60% methane and changing amounts of H₂S.

In principle, the (bio)gas produced is not directly suitable for energy consumption because, amongst others, its relatively low methane content. Additionally, the presence of large amounts of CO₂ and H₂S is undesired in an energy source.

In practice, it is been shown that fermentation of manure alone does not suffice to efficiently convert manure into methane. The addition to the manure of additional nutrient sources such as maize is required to aid the fermentation process.

Further, in practice, it has been shown that only large scale biogas production facilities can be economically exploited for biogas production. On site small scale plants using locally produced biomass are not feasible from an investment and production yield point of view.

US5.529.692 describes a method for biological processing of organic substances and more particularly for anaerobic biological hydrolysis for subsequent biomethanization.

WO2007104418 describes a process and device for obtaining biogas from a fermentation substrate.

EP0661239/US5.591.342 relates to the anaerobic digestion of liquid wastes, comprising an acidogenesis stage followed by a methanogenesis stage. In between the acidogenesis stage and the methanogenesis stage a separation by settling stage is provided to separate treated water from sludge.

WO2011051308 filed in the name of Green Energy Technologies CV is a prior right under Art. 54(3) EPC. This document describes an apparatus and method for conversion of biomass, comprising an acidification reactor, first and second methane synthesis reactors and a nitrification reactor. According to this document, the first synthesis reactor comprises a phase separation device for separating acidified biomass from the acidification reactor into an acidified liquid biomass and an acidified sludge biomass. The first synthesis reactor therefore comprises at least one inlet for receiving acidified biomass and at least two outlets, one for discharging acidified liquid biomass to the second methane synthesis reactor and one for discharging the acidified sludge biomass.

### BRIEF DESCRIPTION

It is an object of the present invention, amongst other objects, to provide a system and method for conversion of biomass into biogas that is able to process biomass in an efficient way.

According to an aspect there is provided an anaerobic biomass processing system for processing biomass using anaerobic digestion, comprising
- an anaerobic acidification reactor, containing acidification bacteria in a suitable environment for acidifying the biomass, and comprising an inlet for biomass and an outlet for acidified biomass,
- an anaerobic settling chamber, comprising an inlet arranged to receive the acidified biomass from the outlet of the anaerobic acidification reactor, and containing methanogenic bacteria in a suitable environment for methanogenesis in biomass in said settling chamber, wherein said biomass is allowed to settle and thereby form a cleared liquid biomass, a sludge biomass and a gaseous biogas effluent, the anaerobic settling chamber comprising a first outlet for the cleared liquid biomass, a second outlet for the sludge biomass and a third outlet for the gaseous biogas effluent, wherein the settling chamber is substantially free from mixing means,
- an anaerobic methane synthesis reactor, containing methanogenic bacteria in a suitable environment for anaerobic microbial conversion of cleared liquid biomass into biogas and processed liquid biomass, comprising an inlet arranged to receive the cleared liquid biomass from the first outlet of the anaerobic settling chamber, a first outlet for biogas and a second outlet for the processed liquid biomass, and mixing means, in particular movable mechanical mixing means, for mixing the biomass in the methane synthesis reactor.

By means of the phrase "inlet arranged to receive" it is meant that the settling chamber and the anaerobic acidification reactor are in liquid connection and that the settling chamber is connected to the anaerobic acidification reactor.

It has been found that such a system allows conversion of biomass in a relatively short time (e.g. 1 to 2 days), in comparison with traditional plants (typically 30 to 40 days). Without wishing to be tied to an explanation, applicants believe that this is at least in part due to the separation of sludge biomass from cleared acidified biomass. Thereby, the methanogenic bacteria in the anaerobic methane synthesis reactor collect to a much lesser extent onto solid particles in the biomass therein. After all, much less solid material is present in the biomass flowing into the anaerobic methane synthesis reactor. This allows better mixing in the biomass. Also, it has been found that such a system has a relatively high efficiency of conversion, methanogenesis already taking place before the actual methanogenese reactor.

In the present invention, the settling chamber, arranged for letting the biomass therein settle, is substantially free from mixing means. This means that there are not provided any purposive means for mixing the contents. It is however not excluded, and hardly avoidable, that gas bubbles generated in the biomass produce a certain mixing effect. However, in an emptied system, no mixing means are discernible. Furthermore, whenever there is an "inlet arranged to receive ... from the ... outlet", this is intended to mean that there is any kind of connection, tubing, conduit, overflow or the like that allows biomass to flow from the first container via the outlet, to the next container, via the inlet.

According to an embodiment the third outlet of the anaerobic settling chamber and the first outlet of the anaerobic methane synthesis reactor are connected to form a combined gas outflow outlet. Both outlets may for instance be connected to respective inlets of a vessel, which comprises a single outlet with a single gas conduit for the combined gas flow, thus providing a simpler supply for further refinement or use.

According to an embodiment the third outlet of the anaerobic settling chamber comprises a pump device, arranged to pump gaseous biogas effluent out of the settling chamber. This acts to extract biogas from the atmosphere above the biomass in the settling chamber. This in turn helps to shift the equilibrium reaction of in particular the methanogenic bacteria in the biomass towards methane production. Since these bacteria need a higher pH, a less acid environment, simply shifting the acidified biomass from the acidification reactor to the settling chamber will not result in a good environment for methane production. However, starting with a good environment, and adding small quantities of the acidified biomass at a time allows the methonagenese reaction to thereby increase the pH that has been lowered by adding the acidified biomass. In this way, the settling chamber can serve a double purpose of settling/clearing the biomass and already producing an amount of methane prior to the methanogenese reactor. The pump device may be any suitable pump device for generating a gas flow in order to remove gas from the anaerobic settling chamber. The pump device may be a fan.

According to an embodiment the anaerobic settling chamber comprises a non-horizontal bottom wall defining a lowest region, wherein the second outlet of the anaerobic settling chamber for the sludge biomass is provided in said lowest region, preferably at or near the lowest point of the bottom wall. Such a non-horizontal bottom wall will help in collecting the sludge mass in one point, the lowest point. By providing the second outlet of the anaerobic settling chamber at or near that lowest point, it is ensured that the sludge biomass is efficiently removed from the anaerobic settling chamber. Herein, "near the lowest point" means that the distance between the lowest point and the second outlet is at most 20%, preferably at most 10%, and most preferably at most 5%, of the largest dimension of the anaerobic settling chamber. Furthermore, the "lowest region" can be defined as the part of the settling chamber that is below the highest level of the non-horizontal bottom wall.

According to an embodiment, the system comprises a pump device arranged to pump sludge biomass out of the settling chamber via the second outlet of the anaerobic settling chamber, such as a screw pump. The pump is provided such that the sludge mass can be pumped out of the anaerobic settling chamber without disturbing the fluid inside the anaerobic settling chamber too much. A screw pump, for instance an Archimedes's screw, is able to pump the sludge biomass out of the anaerobic settling chamber without disturbing the contents of the anaerobic settling chamber too much, i.e. without disturbing the settling process.

According to an embodiment, the system comprises a composting reactor for microbial decomposition of sludge biomass, comprising composting micro-organisms and having a first inlet arranged to receive the sludge biomass from the second outlet of the anaerobic settling chamber, and a second inlet arranged to receive oxygen containing gas, and optionally a third inlet arranged to receive vegetable material and a first outlet for composted biomass. Such an embodiment can provide compost, a readily usable product that can be made from the sludge mass. Optionally, plant or vegetable material can be added for increased production. Examples of vegetable materials may be grass, wood. Addition of vegetable material facilitates the composting process in the composting reactor. Especially since the present invention is suitable for local production, small-scale addition of vegetable material, such as surplus harvest products, plant waste, cuttings or even domestic vegetable material may also be added. Note that a separate first outlet for composted biomass, i.e. compost, is not necessary, as it can be removed manually or with machines, simply from a general top opening of the composting reactor.

In embodiments, the inlet of the composting reactor is at a higher level than the second outlet of the anaerobic settling chamber, and there is provided a connection between the second outlet of the anaerobic settling chamber and the inlet of the composting reactor, which connection comprises a pump device. This can provide a good separation between sludge biomass to be composted in the composting reactor and fluid in the anaerobic settling chamber, without disturbing said settling chamber too much.

According to an embodiment the pump device is a screw pump, which is a useful type for gentle pumping out sludge biomass.

According to an embodiment the first outlet for the acidified liquid biomass comprises a filter device, in particular a so called tilted plate filter device. An example of such a filter device is an arrangement comprising tilted plates being provided in front of the first outlet to prevent relatively big objects from entering the first outlet. Such a filter device is robust and easy to clean in case of an obstruction. Alternatively or additionally to the tilted plate device the filter device can comprise a microfilter. This microfilter keeps bacteria inside the reactor.

According to embodiments, the anaerobic acidification reactor comprises a mixing device, such as a stirring device or a circulation system, and arranged to mix the contents of the acidification reactor. Mixing the contents of the anaerobic acidification reactor increases the efficiency of the chemical processes.

According to an embodiment the mixing device is a circulation system, wherein sensors are provided in the circulation system, such as a pH measuring device, a temperature measuring device, a redox potential measuring device. Preferably, there is provided a control arranged to control the circulation system, circulating contents of the acidification reactor, and the control is arranged to process signals from the sensors for controlling the circulation system. In order to measure accurate pH levels, it is advantageous to position the pH measuring device in a moving fluid. The circulation system provides an advantageous possibility to position a pH measuring device.

In expedient embodiments, a conduit is provided at least between the outlet of the anaerobic acidification reactor and the inlet of the anaerobic settling chamber, wherein the conduit has a conduit inlet in the anaerobic acidification reactor and a conduit outlet in the anaerobic settling chamber, wherein the conduit inlet is provided near a bottom of the reactor and wherein at least a part of the conduit is provided at a level near a top wall of the reactor, preferably the conduit being without pump means.

Herein, the expression "at least between the outlet ... and the inlet..." means that the conduit may run strictly from outlet to inlet, but in most cases the conduit will extend over a certain length into the acidification reactor and/or the settling chamber. Note that the outlet, the inlet, respectively, may be considered a hole in the wall of the respective reactor/chamber. The expression "near a bottom of the reactor" is to be taken as "in the lower half of the reactor, preferably in the lower quarter of the height of the reactor". Similarly, "at a level near a top wall of the reactor" should be interpreted as "positioned with respect of the top wall within at most a quarter of the height of the reactor, preferably at the the level of the top wall". Providing such a conduit, one could call it a siphon, enables the fluid that is taken from the acidification reactor to settle at least partially before entering the settling chamber. After all, the fluid is mixed in the acidification reactor, while it should have as low a velocity as possible in the settling chamber. By using a siphon in which the fluid must move upwardly, it has the chance of settling down. Of course, additionally, the normal function of a siphon is used to transport the fluid from the reactor to the chamber without any pump means, which is preferred, as this ensures as little stirring as possible in the transport. However, the use of additional transport means in the conduit, such as pumps or screws, is not to be excluded.

According to an embodiment, the conduit comprises a first valve with a closeable inlet for flushing fluid, wherein the conduit is provided with a cleaning by-pass conduit connected to at least the closeable inlet, and optionally to both sides of the valve. With this arrangement, it is possible to clean the conduit by flushing. After all, especially in the case that fluid transport is completely without pump means or the like, the conduit is susceptible of contamination or even clogging by parts of the fluid, or particles therein. Optionally, the cleaning by-pass conduit comprises a second valve connected to a water supply to flush the first valve in order to clean the first valve.

According to a non-depicted embodiment the conduit is connected to circulation pump which is provided as part of a mixing device of the anaerobic acidification reactor. This way, fluid may be mixed in the anaerobic acidification reactor and may be pumped from the anaerobic acidification reactor to the anaerobic settling chamber while only requiring the presence of one pump. A valve may be provided to switch the function of the pump from mixing to transferring fluid. The conduit may be connected to mixing channel which is part of the circulation system.

According to an embodiment the anaerobic settling chamber is thermally isolated. By thermally isolating the anaerobic settling chamber, in use, the temperature inside the anaerobic settling chamber may be maintained within the desired operating range (typically 20°C to 60°C), without the need of further heating devices or at least with reduced heating capacity.

In embodiments, the system comprises a container for nutrients for the acidification bacteria, in particular molasses, with a controllable supply means that is connectable to the anaerobic acidification reactor, to control the pH level in said reactor. By thus providing nutrients to the acidification bacteria, the latter may produce more acid, thus lowering the pH to a more agreeable level. Expediently, the nutrients comprise or are molasses, as this product is cheap and readily available, and provides good results in pH control. Alternative nutrients are not excluded, however.

According to an embodiment, the system comprises a pH measuring device, the supply means is connected to the pH measuring device, and the supply means is controlled on the basis of a pH level measured inside the anaerobic acidification reactor by the pH measuring device. By measuring the pH level the pH level can be monitored. If the pH measuring device is furthermore coupled to a pH influencing means, the pH can also be controlled. Such a pH influencing device could simply be a controlled acid and/or base supply, but a safer and cheaper device is or comprises the nutrients/molasses supply as mentioned above. In practice, the pH may become too high, i.e. too basic. If this is measured by the pH measuring device, it then suffices to add nutrients, such as molasses, via the nutrients supply until the pH is measured to be at an acceptable level, or such that digestion by the acidification bacteria will bring it to an acceptable level.

The invention also relates to an anaerobic biomass processing system, comprising a system according to the invention, and further comprising an animal manure storage for storing animal manure and provided with a manure pump, arranged to pump the manure to the inlet of the anaerobic acidification reactor. Although the system described thus far can function very well when the biomass is entered manually, it is particularly advantageous to automate according to the now presented anaerobic biomass processing system. This uses the circumstance that this manure is easily pumpable in most cases, thus alleviating the labour for an operating person. Importantly, it can also ensure that biomass (manure) is added to the anaerobic acidification reactor often, but in small portions. This in turn ensures that the environment in the reactor is relatively stable, which promotes a good processing by the acidification bacteria, and further down the process also the other micro-organisms. Of course, in addition to a manure storage and pump, the system could also comprise other storages and pumps, connected to the anaerobic acidification reactor, such as a molasses storage and pump. The advantages are similar to those for the first mentioned system.

The invention also relates to an animal farm, comprising at least one housing arranged to keep livestock or poultry, and a system according to the invention, in particular an anaerobic biomass processing system as described in the previous paragraph. An important advantage of such an animal farm is that it can process its own waste products, and convert them into useful products. The manure, and possibly vegetable waste, molasses and so on, are turned into a stream of e.g. compost for improving soil structure, biogas for energy production, and liquid manure. Furthermore, since the manure and other waste is processed continuously, there is no need for large storages nor for a correspondingly large transport system for large masses of manure and other waste. Also, the farm as a whole represents an almost closed cycle, which is very useful in general, but in particular for isolated farms, for which transport of waste out, and energy and fertiliser in are relatively expensive. Overall, the transport of manure, or waste in general, fertilizer and energy will become (almost) superfluous.

### SHORT DESCRIPTION OF THE DRAWINGS

Embodiments will now be described, by way of example only, with reference to the accompanying schematic drawings in which corresponding reference symbols indicate corresponding parts, and in which:

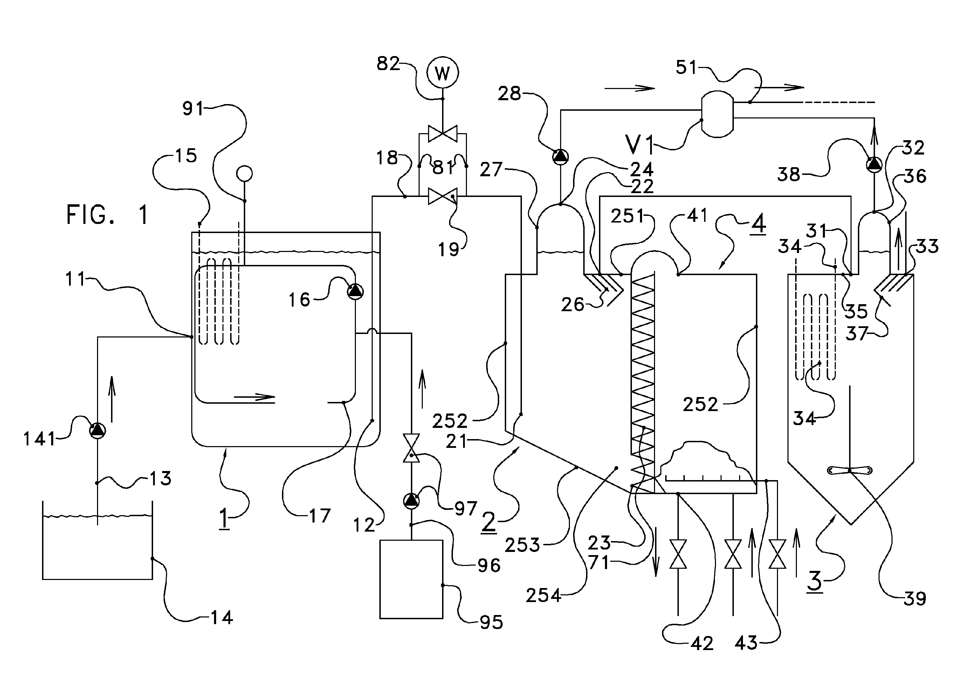

- Fig. 1 schematically depicts an embodiment according to an embodiment.

### DETAILED DESCRIPTION

Fig. 1 shows a system for processing biomass according to an embodiment.

Fig. 1 shows an anaerobic acidification reactor 1 comprising an inlet 11 for biomass and at least one outlet 12 for acidified biomass. The inlet 11 may receive biomass via a manure conduit 13 from a manure storage 14 or the like. A pumping device 141 is provided to transport the biomass from the manure storage 14 to the acidification reactor 1.

The acidification reactor 1 may be a mixed fluid type reactor for microbial hydrolysis and acidification of biomass. The acidification reactor 1 may comprise a heating/cooling device 15 to control the temperature inside the acidification reactor. In use the acidification reactor 1 may be operated at a temperature of 45°C to 70°C. In use, the pH level inside the acidification reactor 1 may be in the range of 3 to 4.5.

Furthermore, a mixing device may be provided to mix the contents of the acidification reactor 1. The mixing device may for instance be a circulation system 16, 17 comprise a circulation pump 16 arranged to pump fluid from a top layer to the bottom layer of the fluid inside the acidification reactor 1 via a mixing channel 17. Of course, other mixing devices may be provided, such as a stirring device.

After acidification of the biomass in the anaerobic acidification reactor 1, the acidified biomass is transported to and discharged in an anaerobic settling chamber 2 as will be described in more detail below, allowing separation of the acidified biomass into a sludge and liquid stream. In a not depicted embodiment a circulation pump may be used to pump the biomass from the anaerobic acidification reactor 1 to the anaerobic settling chamber 2 via conduit 18. Conduit 18 may therefore be connected to mixing channel 17.

Fig. 1 further shows an anaerobic settling chamber 2 comprising an inlet 21 for receiving the acidified biomass from the anaerobic acidification reactor 1. The acidified biomass is allowed to settle inside the anaerobic settling chamber 2. The anaerobic settling chamber 2 is therefore not mixed or stirred. As a result of the settling process an acidified liquid biomass and a sludge biomass is created. The acidified liquid biomass is formed on top of the sludge biomass.

The anaerobic settling chamber 2 comprises a first outlet 22 for the acidified liquid biomass, a second outlet 23 for the sludge biomass. The first outlet 22 is positioned at a level above the second outlet 23. The first outlet 22 may be positioned in a top wall 251 of the anaerobic settling chamber. The first outlet 22 for the acidified liquid biomass may comprise a filter device 26 for instance formed as a plurality of tilted plates.

The anaerobic conditions inside the settling chamber 2 cause formation of methane. The top wall 251 may further comprise an elevated part 27, for instance formed as a domelike structure, in which a third outlet 24 for gaseous biogas effluent is provided. The third outlet 24 is positioned at a higher level than the first outlet 22. The gaseous biogas effluent may comprise methane. The third outlet 24 may comprise a pump device 28 to suck biogas from the anaerobic settling chamber 2. The pump device may be a fan or any other suitable pump device for generating a gas flow. Removal of the methane from the anaerobic settling chamber 2 accelerates the formation process of methane.

The anaerobic settling chamber 2 comprises walls 25 which thermally isolate the contents of the anaerobic settling chamber 2 from the surroundings. The anaerobic settling chamber 2 may comprise the top wall 251, one or more side walls 252 and a bottom wall 253. At least one of these walls 251, 252, 253 may be arranged to thermally isolate the anaerobic settling chamber 2 from it surroundings. The walls 25 may for instance comprise two or more different layers. At least one of the layers, preferably a intermediate layer, may be made of polystyrene. The walls may also comprise an intermediate space filled with air.

The walls may be formed in such a way that the temperature slowly drops until a temperature that matches the temperature of the anaerobic methane synthesis reactor 3, without the need of additional heaters for instance in the anaerobic settling chamber 2.

In use, the anaerobic settling chamber 2 may be operated at a temperature in the range of 20°C to 60°C. In an embodiment, the thermal isolation is sufficient to maintain the temperature within this range, as the acidified liquid biomass received from the anaerobic acidification reactor 1 is at a temperature within the range of 45°C to 70°C.

In use, the anaerobic settling chamber 2 may be at a pH level of 6.5 to 8.

A pH measuring device 91 is provided in circulation system 16, 17. Based on the measured pH level sugar, acid or lye may be added to the mass flowing through the conduit 17. As shown in Fig. 1, a molasses supply 95 may be provided which is connected to conduit 17 via conduit 96. The conduit 96 may comprise a valve 97 to control the inflow of molasses from the molasses supply 95 to the conduit 17.

The redox potential in the anaerobic settling chamber 2 may be in the range of -150 mV to -450 mV.

The bottom wall 253 of the anaerobic settling chamber 2 may be a non horizontal bottom wall 253 defining a lowest region, wherein the second outlet 23 of the anaerobic settling chamber 2 for the sludge biomass is provided at or near the lowest point of the bottom wall 253. The lowest region may be a lowest point or may define a trench. As shown in Fig. 1, the bottom wall 253 is tilted to form a lowest region 254.

Fig. 1 further depicts an anaerobic methane synthesis reactor 3 for anaerobic microbial conversion of acidified liquid biomass into biogas and processed liquid biomass. The methane synthesis reactor 3 may be a mixed fluid type reactor. The anaerobic methane synthesis reactor 3 comprises an inlet 31 for receiving the acidified liquid biomass from the first outlet 22 of the anaerobic settling chamber 2, and a first outlet 32 for gaseous biogas effluent and a second outlet 33 for the processed liquid biomass.

In use, the anaerobic methane synthesis reactor 3 may be operated at a temperature of 20°C to 60°C. The anaerobic methane synthesis reactor 3 may comprise a heating/cooling device 34 in order to maintain the temperature in this range. In use, the anaerobic methane synthesis reactor 3 may be operated with a pH level in the range of 6.5 to 8 and a redox potential of -150mV to -450mV.

The anaerobic methane synthesis reactor 3 may comprise a top wall 35. The inlet of the anaerobic methane synthesis reactor 3 may be provided in the top wall 35. Also, the second outlet 33 may also be provided in the top wall 35.

The top wall 35 may further comprise an elevated part 36, for instance formed as a domelike structure, in which the first outlet 32 for gaseous biogas effluent is provided. The first outlet 32 may comprise a pump device 38 to suck biogas from the anaerobic methane synthesis reactor 3. The pump device 38 may be a fan or any other suitable pump device for generating a gas flow.

The second outlet 33 for the processed liquid biomass may comprise a filter device 37 for instance formed as a plurality of tilted plates.

The third outlet 24 of the anaerobic settling chamber 2 and the first outlet 32 of the anaerobic methane synthesis reactor 3, both for gaseous biogas effluent, may form a combined gas flow 51, for instance for supplying the biogas to a storage tank, further processing equipment or to a transportation conduit. As shown in Fig. 1, both the third outlet 24 of the anaerobic settling chamber 2 and the first outlet 32 of the anaerobic methane synthesis reactor 3 are connected to a vessel V1, from which a combined gas conduit originates.

As shown in Fig. 1, the system further comprises a composting reactor 4 for microbial decomposition of sludge biomass. The compositing reactor 4 comprises an inlet 41 for receiving the sludge biomass from the second outlet 23 of the anaerobic settling chamber 2, and a first outlet 42 for composted biomass. The composting reactor 4 may further comprise a second inlet for receiving vegetable material, such as grass and/or wood.

In use, the composting reactor 4 may be operated at a temperature in the range of 45°C to 75°C. In use, the oxygen concentration may be in the range of 2% - 20%.

In between the inlet 41 of the composting reactor 4 and the second outlet 23 of the anaerobic settling chamber 2 is provided a pump device 71 to overcome the height difference between the inlet 41 of the composting reactor 4 and the second outlet 23 of the anaerobic settling chamber 2. The pump device 71 may be a pump device 71 which doesn't disturb the settling process in the anaerobic settling chamber 2. The pump device 71 may for instance be a screw pump 71.

The composting reactor 4 receives processed sludge biomass from the anaerobic settling chamber 2 and subjects the sludge biomass to the indicated conditions for a period of time sufficient for drying and further digestion, such as for 10 to 30 days.

Controlled oxygen pressure and relatively high temperature ensures efficient composition. In addition, at least partially performing the process at temperatures above 70°C allows for decontaminating the compost of most potential pathogenic micro-organisms. To promote the composting process, an air inlet 43 is provided for introducing compressed air into the compost reactor. A non-shown vertical mixing screw, known in the art, is provided for mixing the compost.

Since the input stream of the composting reactor 4 is low in sulphur, sulphur is removed in the acidification reactor 1, but high in minerals and trace elements, the resulting composted biomass is a high-grade directly usable fertiliser.

The reactors 1, 3 and 4 and the settling chamber 2 are based on microbial conversion, or processing, of biomass. The microorganisms, such as fungi and bacteria, used can be provided by, or present in, the biomass itself, or can be inoculated in the reactors/settling chamber at, for example, start-up of the apparatus. Suitable inoculation cultures can be found in waste and surface water purification installations.

Selection of species of microorganisms is not particularly important. The reaction conditions defined allow the creation of specific environments favouring the growth and/or phenotype of acid producing microorganisms, such as fungi, in the acidification reactor 1, production of methane, for example by bacteria, in the settling chamber 2 and anaerobic methane synthesis reactor 3.

The system may further comprise a controlling device with associated measuring probes to monitor the indicated pHs, temperatures and/or the redox potentials. The system may further comprise means to influence the pH, temperature and/or redox potentials in one or more of the different reactors 1, 2, 4 and the settling chamber 2.

The means may comprise heating/cooling devices for maintaining the temperature in the defined range, pH regulating devices for maintaining the pHs in the defined range, and redox potential regulating devices for maintaining the redox potential in the defined range.

Temperature regulating devices can be heaters providing heat generated or derived from the apparatus itself, or heat from an external source, coolers providing cooling generated or derived from the apparatus itself, or cooling from an external source.

pH regulating devices can be holders comprising sugar, buffer, acid or basic liquid fitted with supply means for introducing the sugar, buffer, acid or basic liquid in the appropriate reactor 1, 3, 4 or settling chamber 2 and/or a transport system controlling the flow of basic or acidic fluids in the apparatus itself. For instance, the acidification reactor 1 provides acidification by acid secretion of microorganisms. However, for example, when the biomass supplied comprises a high nitrogen content, the indicated pH range can be optionally maintained by adding additional sugar or acid to the biomass or into the acidification reactor 1.

Redox potential regulating devices can be holders comprising liquids with a defined redox potential fitted with supply means for introducing the liquids in the appropriate reactor. The point of introduction if preferably into a circulation line 17.

The present system is particularly suitable to process biomass, especially to convert biomass into methane and/or fertilizer, selected from the group consisting of liquid manure, manure, sewage sludge, domestic vegetable waste, agricultural plant residue, domestic plant residue, and combinations thereof.

As indicated above, methane and other high-grade products can be conveniently collected at the outlets of reactors/chamber 1, 2 and 3. According to an embodiment, a system is provided wherein biogas, such as methane, is collected at the outlets of the settling chamber 2 and the anaerobic methane synthesis reactor 3 and compost at the outlet 42 of the composting reactor 4. The liquid flowing from outlet 33 of the anaerobic methane synthesis reactor 3 is very suitable to be used as a liquid fertilizer.

The system as described above provides an efficient conversion of (waste) biomass into valuable products.

The system as described above provides an efficient conversion of (waste) biomass into valuable products. Therefore, according to another aspect, the present invention relates to use of the present system for conversion of biomass. According to a preferred embodiment, the present use results in the conversion of biomass into methane and/or fertilizer and/or compost.

The descriptions above are intended to be illustrative, not limiting. Thus, it will be apparent to one skilled in the art that modifications may be made to the invention as described without departing from the scope of the claims set out below.

## Claims

1. Anaerobic biomass processing system for processing biomass using anaerobic digestion, comprising
- an anaerobic acidification reactor (1), containing acidification bacteria in a suitable environment for acidifying the biomass, and comprising an inlet (11) for biomass and an outlet (12) for acidified biomass,
- an anaerobic settling chamber (2), comprising an inlet (21) arranged to receive the acidified biomass from the outlet (12) of the anaerobic acidification reactor (1), and containing methanogenic bacteria in a suitable environment for methanogenesis in biomass in said settling chamber (2), wherein said biomass is allowed to settle and thereby form a cleared liquid biomass, a sludge biomass and a gaseous biogas effluent, the anaerobic settling chamber (2) comprising a first outlet (22) for the cleared liquid biomass, a second outlet (23) for the sludge biomass and a third outlet (24) for the gaseous biogas effluent, wherein the settling chamber (2) is substantially free from mixing means,
- an anaerobic methane synthesis reactor (3), containing methanogenic bacteria in a suitable environment for anaerobic microbial conversion of cleared liquid biomass into biogas and processed liquid biomass, comprising an inlet (31) arranged to receive the cleared liquid biomass from the first outlet (22) of the anaerobic settling chamber (2), a first outlet (32) for biogas and a second outlet (33) for the processed liquid biomass, and mixing means (39), in particular movable mechanical mixing means, for mixing the biomass in the methane synthesis reactor (3).

2. System according to claim 1, wherein the third outlet (24) of the anaerobic settling chamber (2) and the first outlet (32) of the anaerobic methane synthesis reactor (3) are connected to form a combined gas outflow outlet (51).

3. System according to any one of the preceding claims, wherein the third outlet (24) of the anaerobic settling chamber (2) comprises a pump device (28), arranged to pump gaseous biogas effluent out of the settling chamber (2).

4. System according to any one of the preceding claims, wherein the anaerobic settling chamber (2) comprises a non-horizontal bottom wall (253) defining a lowest region (254), wherein the second outlet (23) of the anaerobic settling chamber (2) for the sludge biomass is provided in said lowest region (254), preferably at or near the lowest point of the bottom wall (253).

5. System according to claim 4, comprising a pump device (71) arranged to pump sludge biomass out of the settling chamber (2) via the second outlet (23), such as a screw pump.

6. System according to any one of the preceding claims, wherein the system comprises a composting reactor (4) for microbial decomposition of sludge biomass, comprising composting micro-organisms and having a first inlet (41) arranged to receive the sludge biomass from the second outlet (23) of the anaerobic settling chamber (2), and a second inlet (43) arranged to receive oxygen containing gas, and optionally a third inlet arranged to receive vegetable material and a first outlet (42) for composted biomass.

7. System according to claim 6, wherein the inlet (41) of the composting reactor (4) is at a higher level than the second outlet (23) of the anaerobic settling chamber (2) and wherein there is provided a connection between the second outlet (23) of the anaerobic settling chamber (2) and the inlet (41) of the composting reactor (4), which connection comprises a pump device (71).

8. System according to any one of the preceding claims, wherein the first outlet (22) for the acidified liquid biomass comprises a filter device (26).

9. System according to any one of the preceding claims, wherein the anaerobic acidification reactor (1) comprises a mixing device, such as a stirring device or a circulation system (16, 17), and arranged to mix contents of the acidification reactor (1).

10. System according to claim 9, wherein the mixing device is a circulation system (16, 17), wherein sensors (91) are provided in the circulation system (16, 17), such as a pH measuring device, a temperature measuring device or a redox potential measuring device.

11. System according to any one of the preceding claims, wherein a conduit (18) is provided at least between the outlet (12) of the anaerobic acidification reactor (1) and the inlet (21) of the anaerobic settling chamber (2), wherein the conduit (18) has a conduit inlet in the anaerobic acidification reactor (1) and a conduit outlet in the anaerobic settling chamber (2), wherein the conduit inlet is provided near a bottom of the anaerobic acidification reactor (1) preferably the conduit (18) being without pump means.

12. System according to claim 11, wherein the conduit comprises a first valve (19) with a closeable inlet (82) for flushing fluid, wherein the conduit (18) is provided with a cleaning by-pass conduit (81) connected to at least the closeable inlet (82), and optionally to both sides of the valve (19).

13. System according to any one of the preceding claims, the system comprising a container (95) for nutrients for the acidification bacteria, in particular molasses, with a controllable supply means (97) that is connectable to the anaerobic acidification reactor (1), to control the pH level in said reactor.

14. System according to claim 13, wherein the system comprises a pH measuring device (91), the supply means (97) is connected to the pH measuring device, and the supply means (97) is controlled on the basis of a pH level measured inside the anaerobic acidification reactor by the pH measuring device (91).

15. Anaerobic biomass processing system, comprising a system according to any of claims 1-14, further comprising an animal manure storage (14) for storing animal manure and provided with a manure pump (141), arranged to pump the manure to the inlet (11) of the anaerobic acidification reactor (1).

16. Animal farm, comprising at least one housing arranged to keep livestock or poultry, and a system according to any of claims 1-15, in particular according to claim 15.
